# EUROPEAN PATENT SPECIFICATION

(11) **EP 0 610 072 B1**
(45) Date of publication and mention of the grant of the patent: **16.04.1997**
(21) Application number: 94300762.5
(22) Date of filing: 02.02.1994
(51) Int. Cl.: A46B 15/00

(54) **Tooth brush**
Zahnbürste
Brosse à dents

(30) Priority: 03.02.1993 BR 9300315
(43) Date of publication of application: 10.08.1994
(73) Proprietor: JOHNSON & JOHNSON CONSUMER PRODUCTS, INC., Skillman, New Jersey 08558-9418 (US)
(72) Inventor: Rangel, Fabio Eduardo França, Jardim Leonidia, Jacarei, SP. (BR); Simionato, Luiz Bellino, Vila Ady-Anna, Sao José Dos Campos, SP. (BR)
(74) Representative: Fisher, Adrian John

(56) References cited:
- GB-A- 2 097 663

## Description

The present invention is in respect to a tooth brush with a means of indication of the adequate time for brushing.

The correct habits for brushing teeth and for the correct time are very important parameters in good buccal hygiene, with the aim of avoiding caries and plaque formation, etc.

While the correct brushing technique depends greatly on the learner, brushing time depends principally on the attention and motivation of the user, and it is this parameter which the invention searches to administer.

There is data demonstrating that brushing teeth for short periods of time is inefficient (SANTOS, V.I.M. dos et alii., Rev. Odont. USP - 4(4);318-322, out/dez 1990, e WADE, A.B. "The prevention of periodontal diseases" Kimpton, London, 1971, pag. 218-23) showing that an indicative means of minimum brushing time is of great value to advise, to teach or even to encourage the user to behave correctly and cautiously in benefit of good oral hygiene.

The state-of-the art technique reveals some attempts to obtain this effect. The documents of the patents JP56100384, US4150106 and FR2591102, for example, discuss dentifrice which change colors after a certain interval of time once brushing has taken place. These documents have no relation with the present invention.

Other indicative means of brushing time are discussed in the state-of-the-art technique, e.g., in the following patent documents:
- DE2918802 - chronometer associated to brushing, set off by the buccal temperature or set off manually to begin timing.
- GB2230942 - dental brush whose handle has a dial with an animated figure set off electrically, indicating the passage of time.
- WO8900302 - an hourglass with children's figures or motifs to mark the brushing time.
- DE3433254 - tooth brush with electric chronometer which emits sound signals after a correct interval of brushing time.
- DE1117180 - tooth brush with electrical device that signals acoustically a correct brushing time, associated to the application of correct pressure.
- US5044037 - tooth brush with a sound generator associated to the handle, which plays music according to the correct brushing time.
- DE3149233 - tooth brush with a device (electrical or spring chronometer, tape recorder or lamp) which remains active for the correct brushing time.
- EP210094 - a support for a tooth brush which registers the time of the brush's absence, associated to the brushing time.

Although efficient for the purpose proposed, such revelations in the state-of-the-art fail because they propose brushing associated to some additional type of device that controls or indicates the passage of time. This implies an elevation in the price for the consumer, due to the inclusion of this additional device and/or because of the increase in manufacturing complexity.

The patent document GB2236071 proposes an alternative to the problem mentioned above, also aiming to indicate the adequate brushing time. A support containing a layer of thermochromic crystal liquid is inserted in the brush handle, so that there is a change in color of this material caused by the heat transmitted by the user's hand, after a certain amount of time of use. The time spent so that such a change (reversible) of color takes place is associated with the brushing time.

Such technology is inefficient for the proposed end because:
- the user may be holding the brush, transmitting heat without necessarily brushing his teeth, for example, while applying paste or for any other reason.
- the way of holding the brush varies from person to person, making the time necessary for the change vary for each user, and in consequence, the brushing time may be too short.
- the way to manufacture such a brush is more complex than a traditional one, because it requires the insertion of a support containing a thermochromic material somewhere carefully defined in the brush handle. If the depth is a little deeper or shorter than necessary, the sensitivity of the material to the heat of the user's hand varies, and the brushing time - already lacking precision, is jeopardized.

Considering the problems encountered in technology, an efficient and low cost tooth brush was developed, associating manufacturing benefits with a precise time change in color, regardless of the user's way of holding it or the time in which the brush, despite being in the user's hand, is not being used to brush his teeth.

It has to do with the tooth brush endowed with thermochromic material dispersed in its head and/or its bristles.

Thus, the following advantages are attained:
- no additional physical operation is necessary in the brush manufacturing, different from those of the known processes in the state of the art (basically injection and bristle placement).
- the perceptible variation of colour during the use of the brush will take place within a period of time substantially constant, because it is the function of the user's mouth temperature, regardless of the way the user holds the brush, or the time he holds it before brushing, as the control is based on the change of colour only in the head and/or bristles.

In a simple way, the functioning of the dental brush of the invention is as follows: the user begins brushing, so the head of the brush remains in the buccal environment whose walls have temperatures close to 36.5°C. The thermochromic material, exposed in a substantially uniform manner to such temperature, starts abandoning the color it possesses at room temperature to assume diverse coloration in the new temperature. The user notices this alteration and understands that, when this occurs, he has brushed his teeth for an adequate minimum time. After brushing has ceased, the brush gradually returns to the room temperature and to the original colour.

It is therefore the objective of this invention to provide a tooth brush as claimed in Claim 1 characterized in that its head and/or bristles comprise a dispersed thermochromic material able to offer a change in colour in a space of time compatible with the adequate brushing time.

Further features of the invention are claimed in Claims 2-10.

The usual meaning employed in the state-of-the-art "head" of the tooth brush is the part which gives support to the bristles, being introduced into the user's mouth during the brushing operation. The said head may have any adequate format, and be made up of any material which - being the head of the brush of the invention, the indicator element of the passage of time - permits the dispersion of the thermochromic material. Examples of adequate materials are: polypropylene, styrene-acrylonitrile, polystyrene, acrylic, cellulose acetate, cellulose propionate, polyamide, mixtures or blends of these materials with each other or with others, etc., preferably polypropylene. Also, as in its usual sense in the technical realm, the body of the brush is made up of two parts, handle and head, which are normally injected as only one piece. The handle of the tooth brush may have any form, it may be distinct from the head of the brush and may be made up of any material suitable for its purpose, for example the ones already described for the head of the brush.

Tooth brushes are known in the realm of technology with integral body and bristles, i.e., made of the same material and injected as one piece. This alternative is also included in the scope of the invention.

Known tooth brush bristles have any suitable forms for the aim in mind, usually filaments gathered in tufts that in turn are fixed to the head of the said brush by staples, anchors, casted to it, etc. The material of the bristles - in the bristles of the brush of the invention are the time indicating element - must be able to contain dispersed thermochromic material, examples of suitable materials are polyamide, polyester, mixtures or blends of these with each other or with other polymers, etc.

The process to obtain the tooth brush of the invention may be any. Preferably, the material of the body of the brush is injected in a mould, the thermochromic material being introduced under the form of a master batch, i.e., a matrix of high concentration of the said thermochromic material in a sufficient quantity so that in the final product, the concentration is adequate to offer a change in colour in the desired time.

In an advantageous way, and to benefit the manufacturing process, the whole body of the tooth brush of the invention (handle + head) is injected with thermochromic material. In this case, one may optionally provide the handle with a coating, cover or paint, so that the change in colour may be perceived only in the head of the brush. Nevertheless, any other processes of manufacturing the body of the tooth brush of the invention are not excluded. For example, dual injection, i.e., a specific one for the head of the brush, containing thermochromic material and another for the handle, without thermochromic material.

Also, within the scope of the invention, are the processes of manufacturing bristles containing thermochromic materials.

Within the many possible variations foreseen by the invention, one may use thermochromic material only for the head of the tooth brush, or only the bristles, or only a group of bristles, or still combinations of the alternatives.

According to the meaning used here, "thermochromic material" is any material, or combinations of materials, possible of reversible color alteration in function of temperature variation.

There are known materials and described in the realm of technology, for example, compound organic donators and receptors of electrons, which form a weak association between each other, which vanishes with the increase in temperature, such as certain aromatic amines and aromatic compounds or substituted nitro aliphatics. Derivatives of ethylene may also be cited, linked to aromatic rings and hetero atoms; cholesteric crystal liquids may also be mentioned made up of layers that move away from each other or approach each other due to the variation of temperature, altering the coloration. There are also other thermochromic materials, as certain inorganic compounds complexed by nickel or copper.

Examples of the thermochromic material adequate to the accomplishment of the invention are the microencapsulated pigments DAITHERMO DR. supplied by Dainichiseika Color & Chemicals Co. Ltd., of Japan.

As reference to the realm of technology about thermochromic materials, without imposing any limitation to the scope of the invention, one may cite "Kirk-Othmer Encyclopedia of Chemical Technology", John Wiley and Sons, 1979, 3° Edition, vol. 6, pag. 130 to 138.

The technician knows how to determine the quantity and quality of the material utilized in the dental brush of the invention in view of the performance to be attained. That is to say, the dental brush of the invention may be projected so that the change of colour occurs in one minute, or two minutes, or any other interval of time, by only controlling in a known way the parameters of the materials and the process of manufacturing. Preferably, the intervals of time spent for the change of colour must occur between 1 and 5 minutes, more preferably, between 1 and 2 minutes.

The following is given as a mere illustration, a practical example of the accomplishment of the invention, without imposing any limitation to the scope of the invention, defined by the claims attached to this report.

In this example of practical accomplishment of the invention, the injection of thermochromic material into the mould is done, so that the whole body (i.e., head + handle) of the dental brush has dispersed thermochromic material.

The equipment utilized was an injection machine Battenfeld, with a closing force equivalent to 140 tons, with a thread profile showing the relation L/D 17, which permits adequate dispersion of the concentrate of the thermochromic material, hereupon denominated master batch, inserted in the process. Associated to the equipment, a balanced dental brush mold was used composed of eight useful cavities, with a total weight of 82.2g and unit weight of each brush body of 9.0g.

The following raw materials were used:
- polypropylene homopolymer with a high rate of fluidity (35g/10min. according to norm ASTM D-1238 with testing conditions of 230°C and 2160g) supplied by PPH Companhia Industrial de Polypropylene S.A. under code PD701.
- thermochromic master batch supplied under code 7560121 by Cromex Resinas Sinteticas, division of ITAP S.A., containing 50% of polypropylene vehicle and 50% of the thermochromic compound DAITHERMO DR-30 Reversible type made by Dainichiseika Colour & Chemicals Co. Ltd. This master batch possesses rates of fluidity 102g/10min. according to norm ASTM already cited, with testing conditions of 230°C and 1200g.

The raw material was mixed manually to obtain a composition of 95% of polypropylene and 5% of thermochromic master batch, and the same was placed in the funnel of the injections machine, utilizing temperatures in the cannon and mouth of the injector between 185 and 195°C. A total cycle of injection of 38 second and time of injection of 7 seconds was utilized with injection pressure of 40kg/cm², counter-pressure of 5kg/cm² and no back pressure.

The resulting handles were then cooled and submitted to the bristling process, utilizing metallic anchors for the bristle tuft fixation in the head of the brush.

A test was then run.

Each one of five users received a brush, and they were instructed to brush their teeth until there was a complete change in color in the head of the brush, from green to yellow, both for intensity of colour as well as the physical area of the head taken up by the new colour.

Initially, the average temperatures of the users' mouths were measured (36.0°C, with a mercury thermometer), and the surfaces of their hands (30.6°C, with a thermopar digital thermometer).

There was no other instruction, for example as to the brushing technique, initial handling of the brush, placement of tooth paste, the act of wetting the paste and/or bristles, etc.

As a result, it was noted that the average time of change in colour of the brush detected by the users was 50 seconds, as the test expected. Photographs taken at the moment each user declared finished, the brushing showed that:
- the head of the brush had totally altered coloration to yellow, both in intensity as well as in physical area, the same for all of the users.
- the body of the brushes presented irregular yellow regions, both in relation to the intensity as well as in relation to the physical area, and in distinct manners for each user.

Therefore, it may be noted that in practice, the way of holding the brush varies greatly from user to user, and consequently, the indication of the brushing time based on the change in the handle colour is not efficient.

As shown in the practical example of the situation above, the present invention is efficient for the indication of time of minimum brushing by the change of the color of the head of the brush (and/or the bristles), without the problems of the previous technique.

## Claims

1. Dental brush characterized in that its head and/or bristles comprise a dispersed thermochromic material, able to offer change of colour in a space of time compatible with the adequate brushing time.

2. Dental brush according to claim 1 characterized in that only the said head comprises the mentioned dispersed thermochromic material.

3. Dental brush according to claim 1 characterized in that only the said bristles comprise the mentioned dispersed thermochromic material.

4. Dental brush according to claim 1 characterized in that only one group of the said bristles comprise the mentioned dispersed thermochromic material.

5. Dental brush according to claim 1 characterized in that the handle of the said brush also comprises the dispersed thermochromic material.

6. Dental brush according to claim 5 characterized in that the said handle has a coating, cover or paint so that the colour is perceived only in the head of the brush.

7. Dental brush according to claim 5 characterized in that the said head and handle are part of one piece.

8. Dental brush according to claim 5 characterized in that the said head, the said handle and the said bristles are part of one piece.

9. Dental brush according to claim 1 characterized in that the said brush is made preferably of polypropylene.

10. Dental brush according to any of the claims 1 to 9 characterized by the fact that the change in colour occurs between 1 and 5 minutes, preferentially between 1 and 2 minutes.

## Patentansprüche

1. Zahnbürste, dadurch gekennzeichnet, daß ihr Kopf und/oder ihre Borsten ein dispergiertes thermochromes Material enthalten, das dazu befähigt ist, innerhalb eines Zeitraums, der mit einer angemessenen Zahnputzzeit im Einklang steht, eine Farbänderung bereitzustellen.

2. Zahnbürste nach Anspruch 1, dadurch gekennzeichnet, daß nur der Kopf das vorerwähnte dispergierte thermochrome Material enthält.

3. Zahnbürste nach Anspruch 1, dadurch gekennzeichnet, daß nur die Borsten das vorerwähnte dispergierte thermochrome Material enthalten.

4. Zahnbürste nach Anspruch 1, dadurch gekennzeichnet, daß nur eine Gruppe der Borsten das vorerwähnte dispergierte thermochrome Material enthält.

5. Zahnbürste nach Anspruch 1, dadurch gekennzeichnet, daß der Griff der Bürste ebenfalls das dispergierte thermochrome Material enthält.

6. Zahnbürste nach Anspruch 5, dadurch gekennzeichnet, daß der Griff einen Überzug, eine Abdeckung oder einen Anstrich aufweist, so daß die Farbe nur am Kopf der Bürste wahrgenommen wird.

7. Zahnbürste nach Anspruch 5, dadurch gekennzeichnet, daß der Kopf und der Griff einstückig ausgebildet sind.

8. Zahnbürste nach Anspruch 5, dadurch gekennzeichnet, daß der Kopf, der Griff und die Borsten einstückig ausgebildet sind.

9. Zahnbürste nach Anspruch 1, dadurch gekennzeichnet, daß die Bürste vorzugsweise aus Polypropylen gefertigt ist.

10. Zahnbürste nach einem der Ansprüche 1 bis 9, dadurch gekennzeichnet, daß die Farbänderung innerhalb von 1 bis 5 Minuten und vorzugsweise innerhalb von 1 bis 2 Minuten erfolgt.

## Revendications

1. Brosse à dents, caractérisée en ce que sa tête et/ou ses poils comprennent une matière thermochromique dispersée pouvant entraîner un changement de couleur dans un intervalle de temps compatible avec le temps de brossage adéquat.

2. Brosse à dents selon la revendication 1, caractérisée en ce que seule ladite tête comprend la matière thermochromique dispersée mentionnée.

3. Brosse à dents selon la revendication 1, caractérisée en ce que seuls lesdits poils comprennent la matière thermochromique dispersée mentionnée.

4. Brosse à dents selon la revendication 1, caractérisée en ce que seul un groupe desdits poils comprend la matière thermochromique dispersée mentionnée.

5. Brosse à dents selon la revendication 1, caractérisée en ce que le manche de ladite brosse comprend également la matière thermochromique dispersée.

6. Brosse à dents selon la revendication 5, caractérisée en ce que ledit manche est muni d'un revêtement, d'un cache ou enduit de peinture de telle sorte que la couleur est perçue uniquement dans la tête de la brosse.

7. Brosse à dents selon la revendication 5, caractérisée en ce que ladite tête et ledit manche sont d'un seul tenant.

8. Brosse à dents selon la revendication 5, caractérisée en ce que ladite tête, ledit manche et lesdits poils forment une seule pièce.

9. Brosse à dents selon la revendication 1, caractérisée en ce que ladite brosse est fabriquée de préférence en polypropylène.

10. Brosse à dents selon l'une quelconque des revendications 1 à 9, caractérisée par le fait que le changement de couleur survient entre 1 et 5 minutes, de préférence entre 1 et 2 minutes.
